# EUROPEAN PATENT APPLICATION

(11) **EP 0 903 151 A1**
(43) Date of publication of application: **24.03.1999**
(21) Application number: 97116494.2
(22) Date of filing: 22.09.1997
(51) Int. Cl.: A61K 45/06, A61K 31/55, A61K 31/415

(54) **Use of combinations comprising non-sedating antihistamines and alpha-adrenergic drugs for the topical treatment of rhinitis/conjunctivitis and cold, cold-like and/or flu symptoms**

(71) Applicant: ASTA Medica Aktiengesellschaft, D-01277 Dresden (DE)
(72) Inventor: Dr. Diez Crespo, Maria del Carmen, 28043 Madrid (ES); Dr.Mainardi, Roberto, 05101-070 Sao Paulo - SP (BR); Prof. Szelenyi, Istvan, D-90571 Schwaig (DE); Dr. Muckenschnabel, Reinhard, D-60388 Frankfurt (DE)

(57) **Abstract**

Pharmaceutical combination, applicable topically, containing a non-sedating antihistamine in combination with an α-adrenergic agonist and optionally conventional physiologically acceptable carriers, diluting agents and auxiliaries substances for the prophylaxis and treatment of allergic and/or vasomotoric rhinitis, conjunctivitis, cold, cold-like and/ or flu symptoms are claimed.

## Description

### Technical field

The present invention relates generally to novel pharmaceutical compositions containing 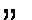 topical decongestants", preferably epinephrine, fenoxazoline, indanazoline, naphazoline, oxedrine, oxymetazoline, phenylephrine, tefazoline, tetryzoline, tramazoline, tymazoline or xylometazoline and a non-sedating antihistamine, applicable topically such as levocabastine, efletirizine, however preferably azelastine. The combinations are useful in the management of allergic and/or vasomotoric rhinitis, conjunctivitis, cold, cold-like and/or flu symptoms.

### Background of the invention

In industrialized countries, more than 10-15 % of the population suffer from allergic rhinitis and/or conjunctivitis. Allergic rhinitis and/or conjunctivitis are type I allergic responses that are mediated by IgE antibodies. As a part of an allergic response to antigen, reaginic antibodies (IgE) are generated and bound to the surface of mast cells and basophils via high affinitiy Fc receptors (FcεRI) that are specific for IgE. Antigen cross-linking the IgE-molekules leads to cellular responses involving release of performed mediators (e.g. histamine), lipid mediator formation and release, and cytokine generation. Mast cells with their mediators can be regarded as central to the initiation and mediation of the early phase of allergic inflammation.

Symptoms of rhinitis are sneezing, itching (nasal irritation), rhinorrhea (nasal secretion) and nasal blockage (congestion). Nasal blockage is the result of the pooling of blood in the capacitance vessels of the mucosa, and to some degree the result of tissue oedema. Patients with allergic conjunctivitis show similar symptoms.

Itching, eye rubbing and tearing are very common, and cause much distress. Conjunctival oedema and hyperemia cause the bulbar surface to take on a 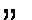 glassy" appearance, with dilated blood vessels.

The common cold is usually not a serious illness but it is highly prevalent, discomforting, and annoying infliction. The term 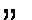 common cold" is applied to minor upper respiratory illnesses caused by a variety of different respiratory viruses, in where rhinoviruses are the major known cause of common cold. Symptoms such as nasal discharge, nasal blockage, and sneezing usually commence on the first day of illness and progress to maximum severity by the 2nd and 3rd day. Along with nasal symptoms may come other symptoms such as cough, burning of the eyes, headache. Fever can also occur.

Antihistamines are generally used in the symptomatic treatment of these disorders. They are effective in reducing itching, sneezing and watery secretion. Based on the ICAM-1(rhinovirus binding site) down-regulating effect of azelastine, it may be particularly useful in the treatment of common cold/flu. Antihistamines are, in general, less effective in the reduction of nasal congestion (blockage). To achieve a further reduction of nasal congestion and ocular oedema, α-adrenergic agonists are often used either alone or in combination with antihistamines.

Non-sedating antihistamines especially such administered topically represent a new generation of histamine H₁ receptor antagonists. They possess strong antagonistic activity at histamine H₁ receptors without causing sedation.
For example, azelastine, a phthalazinone derivative, belongs to this so-called second-generation, non-sedating antihistamines. It is characterized by a long-lasting antiallergic activity and shows a broad spectrum of pharmacologic activities including not only antagonism of histamine H₁-receptors but also inhibition of histamine release following antigen and non-antigen stimuli (Chand, N. et al. Inhibition of allergic and nonallergic leukotriene C4-formation and histamine secretion by azelastine: Implication for its mechanism of action. Int. Arch. Allergy App. Immunol. 90:67-70, 1989). Recently, it has also been demonstrated that topically administered azelastine down-regulates ICAM-1 (intercellular adhesion molecule-1), the binding site for rhinoviruses (Ciprandi, M. D. et al. Topical azelastine reduces eosinophil activation and intracellular adhesion molecule-1 expression on nasal cells: An antiallergic activity. J. Allergy Clin. Immunol. 98: 1088-1096, 1996). Azelastine is free of cardiovascular side effects. Because the drug is administered topically, the plasma levels following topical azelastine application are extremely low, even if it is overdosaged. By contrast, oral formulations containing antihistamines of the second generation such as terfenadine, astemizole, loratadine may cause cardiovascular side effects (e.g. tachyarrhythmias) when the recommended dosage is not kept.

With regard to allergic diseases and common cold, either topical or oral α-adrenergic agonists are used as disclosed in WO 94/ 08551. Oral decongestants (e.g. pseudoephedrine, phenylephrine, phenylpropanolamine as disclosed in, for example, WO 92/ 04021, WO 92/ 04022, WO 94/ 08550, WO 94/ 14449 and WO 95/ 23591) carry the risk of inducing systemic adverse effects such as tachycardia, increased blood pressure, and CNS stimulation (e.g. insomnia). Topical α-adrenergic agonists such as epinephrine, fenoxazoline, indanazoline, naphazoline, oxedrine, oxymetazoline, phenylephrine, tefazoline, tetryzoline, tramazoline, tymazoline, xylometazoline are used as local decongestants in patients with allergic or vasomotor rhinitis, conjunctivitis or with upper respiratory infections (e.g. common cold, flu). α-Adrenergic drugs probably decrease resistance to airflow by decreasing the volume of the nasal mucosa. This may occur by activation of α-adrenergic receptors in venous capacitance vessels in nasal tissues. Topical decongestants are particularly useful because of their more selective site of action. Although their topical administration is associated with few systemic adversed effects, prolonged use may result in rebound congestion and worsening of symptoms. Therefore, the use of formulations containing a nasal vasoconstrictor compound is not recommended longer than 7-10 days. In addition, the recommended daily dosage should not be exceeded.

For oral use, combinations containing antihistamines and α-adrenergic agonists are widely used both for the treatment of allergic rhinitis and common cold as disclosed in WO 88/ 09656, WO 94/ 14476, WO 94/ 25009 and WO 95/ 07103. It has been demonstrated that patients suffering from common cold or allergic rhinitis have benefit from oral antihistamine + decongestant therapy (Anonymus. The management of hay fever. Drug Ther. Bull. 23:25-27, 1985; Berkowitz, R. B. et al. The effectiveness of the nonsedating antihistamine loratadine plus pseudoephedrine in the symptomatic management of the common cold. Ann. Allergy 63:336-339, 1989). Topical α-adrenergic agonists in combination with antihistamines are administered in which sedating antihistamines(e.g. antazoline) are used. Topical formulations containing antihistamines of the second generation and α-adrenergic agonists are not available and unknown at the moment. Topical decongestants (α-adrenergic agonists) provide quick relief of nasal or ocular oedema. Therefore, they could improve the therapeutic effectiveness of topically applied antihistamines such as azelastine.

Formulations for the treatment of cold, cold-like and/or flu symptoms often contain antihistamines and decongestants. Until now, only oral combination preparations are on the market. The therapeutic activity of a non-sedating antihistamine, applied topically as for instance azelastine, a histamine H₁-receptor antagonist can effectively be supported by α-adrenergic agonists without increasing the risk of adverse effects.

### Summary of the invention

It is therefore an object of the present invention to provide pharmaceutical combinations of matter, applicable topically, comprising an effective amount of a non-sedating antihistamine, preferably acrivastine, antazoline, astemizole, azelastine, cetirizine, ebastine, efletirizine, epinastine, fexofenadine, loratidine, levocabastine, mizolastine, oxatomide, setastine, temelastine or terfenadine in combination with an α-adrenergic agonist, preferably epinephrine, fenoxazoline, indanazoline, naphazoline, oxedrine, oxymetazoline, phenylephrine, tefazoline, tetryzoline, tramazoline, tymazoline or xylometazoline and further optionally including pharmaceutically acceptable carriers and/or diluting agents or auxiliary substances therefor.

It is an additional object of the present invention to provide a method for the prophylaxis and treatment of allergic and/or vasomotoric rhinitis, conjunctivitis, cold, cold-like and/or flu symptoms in a mammalian organism in need of such treatment by topical administering a safe and effective amount of a combination comprising a non-sedating antihistamine, preferably acrivastine, antazoline, astemizole, azelastine, cetirizine, ebastine, efletirizine, epinastine, fexofenadine, loratidine, levocabastine, mizolastine, oxatomide, setastine, temelastine or terfenadine with an α-adrenergic agonist, preferably epinephrine, fenoxazoline, indanazoline, naphazoline, oxedrine, oxymetazoline, phenylephrine, tefazoline, tetryzoline, tramazoline, tymazoline or xylometazoline.

It is another object of the present invention to provide suitable dosage unit forms of a non-sedating antihistamine, preferably acrivastine, antazoline, astemizole, azelastine, cetirizine, ebastine, efletirizine, epinastine, fexofenadine, loratidine, levocabastine, mizolastine, oxatomide, setastine, temelastine or terfenadine in combination with an α-adrenergic agonist, preferably epinephrine, fenoxazoline, indanazoline, naphazoline, oxedrine, oxymetazoline, phenylephrine, tefazoline, tetryzoline, tramazoline, tymazoline or xylometazoline, adapted for convenient topical administration, for instance, in the form of sprays or drops.

### Detailed description of the invention

The antiallergic component in the pharmaceutical combination of the present invention includes a non-sedating antihistamine applied topically as for instance acrivastine, antazoline, astemizole, azelastine cetirizine, ebastine, efletirizine, epinastine, fexofenadine, loratidine, levocabastine, mizolastine, oxatomide, setastine, temelastine or terfenadine or a pharmaceutically acceptable salt thereof. Azelastine is a histamine H₁-receptor antagonist of the second generation without sedating effect.
The concentration of the antihistaminic component of the present invention may range from 0.001% to 0.5%.

In addition to the antihistaminic component the pharmaceutical combinations of the present invention comprise a topical decongestant, preferably epinephrine, fenoxazoline, indanazoline, naphazoline, oxedrine, oxymetazoline, phenylephrine, tefazoline, tetryzoline, tramazoline, tymazoline or xylometazoline, or a pharmaceutically acceptable salt thereof.
The concentration of α-adrenergic agonists in the combination may range from 0.001% to 0.2%.
The concentration of phenylephrine may however range from 0,01% to 15%, its preferred concentration is between 0.1% to 2%.

The preferred concentrations are for the antihistaminic component 0.05% - 0.1% and for α-adrenergic agonists 0.05% - 0.1%, with exception of phenylephrine (its preferred concentration see above).

The active ingredients are administered topically as a mixture containing pharmaceutical diluents, excipients or carrier which are consistent with conventional pharmaceutical practices.

As representative suitable formulations consistent with the objects, features and advantages of the invention, the following examples are provided.

### Dosages:

The pharmaceutical combination is administered as a rule, for nasal application :1 puff/nostril twice daily; maximum daily dose 3 puffs/nostril and for ocular application: 1 drop/eye twice daily, maximum daily dose 3-6 drops/eye.

The compositions for topical applications can be formulated in various pharmaceutically acceptable forms e.g. as nasal sprays or nasal drops or as eye drops.
Besides the active ingredients the compositions of the present invention may further comprise various ingredients such as antimicrobial preservatives, tonicity agents, thickening agents, excipients for pH-adjustment or buffer systems.

For example antimicrobial preservatives can include: benzalkonium chloride, chlorobutanol, thiomersal, methylparaben, propylparaben, sorbic acid, edetate disodium, phenylethanol, chlorhexidine HCl, chlorhexidine acetate, chlorhexidine digluconate, cetylpyridinium chloride / bromide, chlorocresol, phenylmercuric acetate, phenylmercuric nitrate, phenylmercuric borate, phenoxyethanol.
For preservation preferably a combination of edetate disodium and benzalkonium chloride is used. Edetate disodium is used in concentrations of 0.05 - 0.1 % and benzalkonium chloride in concentrations of 0.005 - 0.05 %.

Suitable excipients which can be used to adjust tonicity or osmolality can include: sodium chloride, potassium chloride, mannitol, glucose, sorbitol, glycerol, propylene glycol. In general these agents are used in concentrations of about 0.1 to 10 %.

The compositions often contain thickening agents to increase the viscosity and prolong contact of the drug with the tissue.

Such thickening agents may be: methylcellulose, hydroxypropyl methylcellulose, hydroxyethylcellulose, sodium carboxymethylcellulose, polyvinyl alcohol, polyvinyl pyrrolidone, polyacrylates, polyacrylamide, dextran, gellan gum (Gelrite®), poloxamere, cellulose acetate phthalate.

The compositions of the present invention also include pharmaceutically acceptable buffers sufficient to adjust and maintain the pH in the range of about 4 to 8, preferably about 5.5 to 7.5.
Suitable buffers are citrate, phosphate, tromethamine, glycine, borate, acetate. These buffers can be built from substances like citric acid, monosodium phosphate, disodium phosphate, glycine, boric acid, sodium tetraborate, acetic acid, sodium acetate. Also other excipients can be used for pH-adjustment like hydrochloric acid or sodium hydroxide.

Further details of this invention are given by the following examples which should not be limitations to the scope of the present invention.

### Example 1

### Nasal spray or nasal drops containing Azelastine HCl (0.1%) and Oxymetazoline HCl (0.05%)

10 ml solution contain:

| | |
|---|---|
| Azelastine Hydrochloride | 0.01000 g |
| Oxymetazoline Hydrochloride | 0.00500 g |
| Hydroxypropyl Methylcellulose | 0.01000 g |
| Edetate Disodium | 0.00500 g |
| Benzalkonium Chloride | 0.00125 g |
| Citric Acid anhydrous | 0.00438 g |
| Disodium Phosphate Dodecahydrate | 0.04655 g |
| Sorbitol Solution 70 % | 0.60000 g |
| Purified Water to | 10.0 ml |

### Example 2

### Eye drops containing Azelastine HCl (0.05%) and Tetryzoline HCl (0.05%)

| | |
|---|---|
| Azelastine Hydrochloride | 0.00500 g |
| Tetryzoline Hydrochloride | 0.00500 g |
| Hydroxypropyl Methylcellulose | 0.01000 g |
| Edetate Disodium | 0.00500 g |
| Benzalkonium Chloride | 0.00125 g |
| Sodium Hydroxide | q.s. pH 6.0 |
| Sorbitol Solution 70% | 0.66666 g |
| Water for Injections to | 10.0 ml |

### Example 3

### Nasal spray or nasal drops

See example 1 but with 0.1% Xylomethazoline HCl instead of 0.05% Oxymethazoline HCl

### Example 4

### Eye drops

See example 2 but with 0.1% Naphazoline HCl instead of 0.05% Tetryzoline HCl

### Example 5

### Nasal spray or nasal drops

See example 1 but with 0.05% Naphazoline HCl instead of 0.05% Oxymethazoline HCl

### Example 6

### Nasal spray or nasal drops

See example 1 but with 0.1264 % Tramazoline HCl instead of 0.05 % Oxymethazoline HCl

### Example 7

### Eye drops

See example 2 but with 0.0632% Tramazoline HCl instead of 0.05% Tetryzoline HCl

### Preparation of eye drops (Example 2, 4 and 7):

Prepare 45.0 kg of water for injections in a suitable container and dissolve therein while stirring:
the active principles, edetate disodium, benzalkonium chloride, hydroxypropyl methylcellulose and sorbitol solution. Fill up the solution with water for injection to 49.5 litres. Adjust the pH of the solution with sodium hydroxyde solution 1N to pH 6. Fill up the solution with water for injections to get 50.0 litres and stir. Sterile-filter the solution through a membrane filter, pore size 0.2 µm. Fill the solution aseptically into sterilised bottles.

### Preparation of nasal sprays or nasal drops (Example 1, 3, 5 and 6)

Prepare 96.5 kg of purified water in a suitable container and dissolve therein while stirring:
the active principles, edetate disodium, sorbitol solution, benzalkonium chloride disodium phosphate dodecahydrate, citric acid anhydrous and hydroxypropyl methylcellulose. Fill up the solution to 100 litres and stir. Filter the solution through a membrane filter of pore size 0.2 µm and fill it into bottles.

## Claims

1. A pharmaceutical combination of matter for use in the treatment of allergic and/or vasomotoric rhinitis, conjunctivitis, cold, cold-like and/ or flu symptoms, and adapted for unit dosage topical administration, said combination comprising
a) an effective amount of a non-sedating antihistamine or a pharmaceutically acceptable salt thereof,
b) an effective amount of an α-adrenergic agonist or a pharmaceutically acceptable salt thereof and
c) conventional physiologically acceptable carriers and/ or diluting agents or auxiliary substances.

2. The pharmaceutical combination as defined by claim 1, said non-sedating antihistamines comprising acrivastine, antazoline, astemizole, azelastine, cetirizine, ebastine, efletirizine, epinastine, fexofenadine, loratidine levocabastine, mizolastine, oxatomide, setastine, temelastine or terfenadine.

3. The pharmaceutical combination as defined by claim 1, said α-adrenergic agonists comprising epinephrine, fenoxazoline, indanazoline, naphazoline, oxedrine, oxymetazoline, phenylephrine, tefazoline, tetryzoline, tramazoline, tymazoline or xylometazoline.

4. The pharmaceutical combination as defined by claim 1 and 2, comprising 0.001% to 0.5% of the antihistaminic component.

5. The pharmaceutical combination as defined by claims 1, 2 and 4, comprising preferably 0.05% to 0.1% of the antihistaminic component.

6. The pharmaceutical combination as defined by claims 1 and 3, comprising 0.001% to 0.2% of α-adrenergic agonist, with the exception of phenylephrine.

7. The pharmaceutical combination as defined by claims 1, 3 and 6, comprising preferably 0.05% to 0.1% of α-adrenergic agonist, with the exception of phenylephrine.

8. The pharmaceutical combination as defined by claims 1 and 3, comprising 0.01% to 15% of phenylephrine.

9. The pharmaceutical combination as defined by claims 1, 3 and 8, comprising preferably 0.1% to 2% of phenylephrine.

10. The pharmaceutical combination as defined by claims 1 to 9 in topical dosage form.

11. The pharmaceutical combination as defined by claims 1 to 10 in topical dosage spray form.

12. The pharmaceutical combination as defined by claims 1 to 10 in topical dosage drop form.

13. Use of a pharmaceutical combination of matter, comprising
a) an effective amount of a non-sedating antihistamine or a pharmaceutically acceptable salt thereof,
b) an effective amount of an α-adrenergic agonist or a pharmaceutically acceptable salt thereof and
c) conventional physiologically acceptable carriers and/ or diluting agents or auxiliary substances,
for the production of a medicament for the prophylaxis and treatment of allergic and/ or vasomotoric rhinitis, conjunctivitis, cold, cold-like and/ or flu symptoms by topical administration.

14. Use of a pharmaceutical combination of matter as defined by claim 13, said non-sedating antihistamine comprising acrivastine, antazoline, astemizole, azelastine, cetirizine, ebastine, efletirizine, epinastine, fexofenadine, loratidine, levocabastine, mizolastine, oxatomide, setastine, temelastine or terfenadine, for the production of a medicament for the prophylaxis and treatment of allergic and/or vasomotoric rhinitis, conjunctivitis, cold, cold-like and/or flu symptoms, by topical administration.

15. Use of a pharmaceutical combination of matter as defined by claim 13, said α-adrenergic agonist comprising epinephrine, fenoxazoline, indanazoline, naphazoline, oxedrine, oxymetazoline, phenylephrine, tefazoline, tetryzoline, tramazoline, tymazoline or xylometazoline, for the production of a medicament for the prophylaxis and treatment of allergic and/ or vasomotoric rhinitis, conjunctivitis, cold, cold-like and/ or flu symptoms, by topical administration.

16. Medicaments for topical administration having action against allergic and/ or vasomotoric rhinitis, conjunctivitis, cold, cold-like and/ or flu symptoms comprising
a) an effective amount of a non-sedating antihistamine or a pharmaceutically acceptable salt thereof,
b) an effective amount of an α-adrenergic agonist or a pharmaceutically acceptable salt thereof and
c) conventional physiologically acceptable carriers and/ or diluting agents or auxiliary substances.

17. Medicaments as defines by claim 16, said non-sedating antihistamine comprising acrivastine, antazoline, astemizole, azelastine, cetirizine, ebastine, efletirizine, epinastine, fexofenadine, loratidine, levocabastine, mizolastine, oxatomide setastine, temelastine or terfenadine.

18. Medicaments as defined by claim 16, said α-adrenergic agonist comprising epinephrine, fenoxazoline, indanazoline, naphazoline, oxedrine, oxymetazoline, phenylephrine, tefazoline, tetryzoline, tramazoline, tymazoline or xylometazoline.
